# EUROPEAN PATENT SPECIFICATION

(11) **EP 1 647 288 B1**
(45) Date of publication and mention of the grant of the patent: **23.12.2009**
(21) Application number: 05022468.2
(22) Date of filing: 14.10.2005
(51) Int. Cl.: A61L 27/36

(54) **Method and apparatus for repairing bone**
Verfahren und Gerät zur Knochenwiederherstellung
Méthode et appareil pour la reconstruction osseuse

(30) Priority: 14.10.2004 US 964950
(43) Date of publication of application: 19.04.2006
(73) Proprietor: Biomet Manufacturing Corp., Warsaw, IN 46582 (US)
(72) Inventor: Kumar, Mukesh, Warsaw, Indiana 46582 (US); Hamman, Ned M., Leesburg, Indiana 46538 (US); Leach, Michael, Warsaw, Indiana 46582 (US)
(74) Representative: Albrecht, Thomas

(56) References cited:
- EP-B1- 1 301 222
- WO-A-97/25941
- US-A1- 2002 076 429
- US-B1- 6 458 375

## Description

### INTRODUCTION

This invention relates to a bone repairing composition, methods of production and use thereof In particular, this invention relates to formed compositions useful in repairing osseous defects which may be inserted into the defect without preparation or manipulation.

A bone repairing composition or filler may be used to correct defects caused by trauma, pathological disease, surgical intervention or other situations where defects need to be managed in osseous surgery. Because defects arc visually jagged or irregularly shaped, it may be important to have the bone filler of an appropriate composition to facilitate placement of the filler into the surgical site. The surgeon may trowel the filler into the injury site and use his or her fingers and/or suitable instruments to shape it into the proper configuration.

Bone reconstruction may be performed with various pastes, gels or putty-like materials containing a natural collagen or human cadaveric donor bone base. Preferably, compositions are prepared from demineralized allograft bone matrix (DBM) that is taken from cadavers. The sterile DBM is available in cubes, shavings or powder and is freeze-dried. Because the DBM is dry and difficult to manipulate, it may be made flowable or malleable with the addition of a wetting agent. The patient's blood has been used to mix the bone, bone powder or collagen because blood offers the benefits of being available at the operative site, is non-immunogenic to the patient and contains proteins, monosaccharides, polysaccbarides and glucoronic acid which increase the healing process and regeneration of bone. Other wetting agents include monosaccharides, disaccharides, water dispersible oligosaccharides, polysaccharides, low weight organic solvents, including glycerol, polyhydroxy compounds, such as mucopolysaccharide or polyuronic acid and various aqueous solutions. (See, e.g., U.S. Patent Nos. 5,290,558, O'Leary et al., issued March 1, 1994; 5,073,373, O'Lcary et al., issued December 17, 1991; 5,314,476, Prewett, et al., issued May 27, 1994; 5,507,813, Dowd, et al., issued April 16, 1996; 4,191,747, Scheicher, issued March 4, 1980; and 4,172,128, Thiele, et al., issued October 23, 1979.) Compounds like GRAFTON® (Available from Osteotech, Inc., Eatontown, New Jersey, USA), a glycerol based, non-cross linkable composition and collagen suspended in various inert polyhydroxy compounds, arc also used to make the demineralized bone malleable. Regardless of the exact components, a primary goal in bone reconstruction is that the filler be highly effective in inducing bone formation, become an integrated fixture at the application site and not become dislodged.
US 6,458,375 B1 discloses a malleable paste with allograft bone reinforcement. The '375 patent provides a "viscous formulation ... designed to present the bone matrix, and its bone morphogenetic proteins (BMP)" (column 5, lines 43-45). Various high molecular weight hydrogels arc used for the carrier and the viscosity of the hydrogels increases packing of the paste in the wound (column 6, lines 23-32). The flowable and malleable properties of this composition allow the paste to be dispensed into the defect site and subsequently shaped (column 10, lines 25-28).
US 2002/0076429 A1 discloses a bone paste. The carrier is formed by treating the starting bone material with acetic acid and pepsin (paragraph [0050]). Bone powder is then mixed with the carrier to provide the final, flowable bone paste composition having a viscosity of about 3600 centipoises or higher at 44°C (paragraph [0059]). The flowable bone paste composition is extruded from a syringe and into the defect site (paragraph [0059]). The formulation of the bone paste allows the paste to solidify when the temperature of the composition is raised above its liquefication temperature (about 45°C).
WO 07/25941 discloses a process for fabricating a shaped material from demineralized bone particles. A liquid bone slurry is prepared from demineralized elongate bone particles (2 mm up to 200 mm long) and a liquid carrier such as water, glycerol, or an aqueous solution, such as physiological saline (page 5, lines 12-19 and page 9, lines 18-26). The liquid bone slurry is then placed into an apparatus and is pressed to expel the liquid component and form the bone particles into the desired shape (page 10, lines 1-12). The pressed bone sheet is then prepared for a warming step to dry the sheet and is subsequently freeze-dried (page 12, lines 12-24 and page 13, lines 11-24). The warming step allows the shaped material to maintain its cohesive properties (page 15, lines 1-5).
EP 1 301 222 B1 relates to a porous three-dimensional osteoimplant comprising a low density coherent matrix of bone particles and to a method for making the osteoimplant. The osteoimplant is claimed to maintain its shape and cohesiveness upon absorption of fluid. The porous, absorbent osteoimplant can possess any desired shape, for example, square or rectangular blocks, cylinders, wedges, and the like, and is provided with one or more cavities which can be filled with an osteogenic material which promotes and/or accelerates new bone formation at the site of implantation.

Many compositions known in the art are difficult to handle and shape. The malleable filler must be molded by the surgeon to fit into the proper configuration of the defect site. Even when the surgeon uses great care to mix the paste or gel and sculpt a form, there may be a risk that the implant will become dislodged and carried away by body fluids. Subsequently, these compositions may not be suitable for large defects.

It would be advantageous to provide a bone repairing composition that is non-immunogenic, osteogenic, is easily placed into injury sites, adheres to the injury sites and is not easily displaced by bodily or other fluids. It would also be advantageous for the bone repairing composition to be ready to use in preformed shapes or universally sized patches or sheets, thus eliminating the need for significant sculpting and manipulation of the composition in the operating room. It would also be advantageous to provide a system to efficiently hydrate the compositions.

### SUMMARY

The present invention provides a rigid formed composition for application to a bone surface of a human or animal subject, comprising:
(a) a bone material; and
(b) a carrier comprising denatured demineralized bone;
wherein the composition is formed into a rigid shape suitable for administration to the bone,
wherein the carrier is made by a process comprising:
(a) mixing demineralized bone with water; and
(b) heating the mixture of demineralized bone and water under pressure,
wherein the heating comprises autoclaving the mixture.

Various methods are also provided for making formed compositions for application to a bone surface of a human or animal subject, comprising:
(a) mixing a demineralized bone and water,
(b) heating the mixture of demineralized bone and water to form a carrier;
(c) mixing the carrier with a bone material to form a moldable composition;
(d) molding the moldable composition; and
(e) lyophilizing the moldable composition to produce a rigid formed composition having a shape suitable for administration to the bone.

The invention also provides the use of the rigid formed composition for the preparation of a bone repairing composition useful in repairing osseous defects by a method of augmenting bone at a site in need thereof in a human or animal subject, comprising:
(a) adding water to a dried composition comprising:
   (i) a bone material; and
   (ii) a carrier comprising denatured demineralized bone; wherein said composition is formed into a rigid shape suitable for administration to said bone; and
(b) applying said rigid formed composition to said site.

The compositions and methods of this invention provide benefits over methods, compositions and apparatus among those known in the art. Such benefits may include one or more of affording a graft material that is not easily dislodged from the site to which it is implanted, even in the presence of body fluids and after the passage of time, enhanced strength, and resistance to dissolution by blood or other fluids and easy hydration. Further areas of applicability will become apparent from the detailed description provided hereinafter.

### DETAILED DESCRIPTION

The compositions of this invention comprise a bone material and a carrier component. The following definitions and non-limiting guidelines must be considered in reviewing the description of this invention set forth herein.

The headings (such as "Introduction" and "Summary") and sub-headings (such as "Methods of Augmenting a Bone Site" or "Hydration Apparatus") used herein are intended only for general organization of topics within the disclosure of the invention, and are not intended to limit the disclosure of the invention or any aspect thereof. In particular, subject matter disclosed in the "Introduction" may include aspects of technology within the scope of the invention, and may not constitute a recitation of prior art. Subject matter disclosed in the "Summary" is not an exhaustive or complete disclosure of the entire scope of the invention or any embodiments thereof. Classification or discussion of a material within a section of this specification as having a particular utility (e.g., as being a "carrier" or a "bone building" ingredient) is made for convenience, and no inference should be drawn that the material must necessarily or solely function in accordance with its classification herein when it is used in any given composition.

The citation of references herein does not constitute an admission that those references are prior art or have any relevance to the patentability of the invention disclosed herein. Any discussion of the content of references cited in the Introduction is intended merely to provide a general summary of assertions made by the authors of the references, and does not constitute an admission as to the accuracy of the content of such references.

The description and specific examples, while indicating embodiments of the invention, are intended for purposes of illustration only and are not intended to limit the scope of the invention. Moreover, recitation of multiple embodiments having stated features is not intended to exclude other embodiments having additional features, or other embodiments incorporating different combinations the stated of features. Specific examples are provided for illustrative purposes of how to make and use the compositions and methods of this invention and, unless explicitly stated otherwise, are not intended to be a representation that given embodiments of this invention have, or have not, been made or tested.

As used herein, the words "referred" and "preferably" refer to embodiment of the invention that afford certain benefits, under certain circumstances. However, other embodiments may also be preferred, under the same or other circumstances. Furthermore, the recitation of one or more preferred embodiments does not imply that other embodiments arc not useful, and is not intended to exclude other embodiments from the scope of the invention. As referred to herein, all compositional percentages are by weight of the total composition, unless otherwise specifies.

As used herein, the word "include," and its variants, is intended to be non-limiting, such that recitation of items in a list is not to the exclusion of other like items that may also be useful in the materials, compositions, devices, and methods of this invention.

### Materials

The present invention provides a formed composition for application to a bone surface of a human or animal subject, comprising:
(a) a bone material; and
(b) a carrier comprising denatured demineralized bone;
wherein said composition is formed into a shape suitable for administration to said bone. As referred to herein, a "formed" composition has a non-random shape, preferably of a size and dimension suitable for implantation to the site of a bone surface. Formed compositions may be of any of a variety of shapes, including cubes or other blocks, sheets, rods, rings, and discs. In various embodiments the shapes may be specifically formed for a desired end-use application, as a site-specific pre-form.

Bone used in embodiments of this invention may be obtained from cortical, cancellous and/or corticocancellous bone. (See, e.g., U.S. Patent. 5,507,813, Dowd, et al., issued April 16, 1996.) Preferably, the bone is autologous bone or donated from a single member of the same species as the patient to reduce or prevent an immunogenic response. However, bone from multiple donors may be used in the compositions.

The "bone material" component of the present invention is selected from bone powder, bone chips, bone shavings and mixtures thereof. In a preferred embodiment, the bone material is dried demineralized bone powder. Suitable drying technique include freeze drying, vacuum drying, air drying, temperature flux drying, molecular sieve drying and other appropriate techniques. Preferably, the bone material comprises freeze dried bone. As used herein, the term "freeze dried" or "lyophilization" and variants thereof, means the process of isolating a solid substance from solution by freezing the solution and evaporating the ice under a vacuum. The dried bone material has a final moisture level of about less than 6% as recommended by the American Association of Tissue Banks. As used herein, the term "demineralized" and variants thereof, means a loss or decrease of the mineral constituents or mineral salts of the individual tissues or bone relative to their natural state. Preferably, the demineralized bone has a calcium concentration of about 1%. The demineralized bone powder has a particle size of less than about 1500 microns, more preferably less than about 1000 microns and more preferably, less than about 850 microns. In another preferred embodiment, the demineralized bone material has a particle size less than about 710 microns.

In various embodiments, the bone material may additionally comprise bone chips. The bone chips may be natural or demineralized. The bone chips range from 750 to 2000 microns, preferably from 750 to 1500 microns.

The carrier component is comprised of demineralized bone and an aqueous solution. The carrier component particles sizes arc less than 1500 microns, more preferably less than 1000 microns and more preferably, less than 850 microns. In another preferred embodiment, the demineralized bone material has a particle size less than 710 microns.

The carrier component comprises from 0.2% to 40% of demineralized denatured bone, by weight of the carrier, more preferably from 0.5% to 25% and more preferably, from 10% to 20%. An aqueous solution such as water or saline makes up the remainder of the carrier component.

In various embodiments, autoclaving the carrier component results in the bone and water or saline mixture forming a gel or having a gel like consistency. As used herein, "autoclaving", and its variants, refers to a thermal procedure, such as that used for sterilization, where the solution is placed in a sealed chamber and subjected to high temperature and pressure. Specific autoclaving methods among those useful herein arc further described in the methods section below. Methods along those useful herein are also disclosed in U.S. Patent No. 6,576,249, Gendler et al., issued June 10, 2003.

In various embodiments, the formed product comprises from 10% to 40% bone material, preferably from 20% to 30%. The carrier component comprises from 60% to 90%, preferably from 70% to 80% of the formed composition.

The relative percentages of the bone material and carrier component may vary based on the amounts of each component used and the addition of other materials such as bone building materials. As used herein, a "bone building material" is a compound that stimulates the growth of bone to replace the bone repairing composition. "Bone building material" includes calcium containing materials, nutrient factors, bone morphogenic proteins, growth factors, antimicrobials, anti-inflammatory agents, blood products and mixtures thereof. (See, e.g., U.S. Patent No. 6,180,606, Chen, et al., issued January 30, 2001.) Depending on the bone building material or materials selected, the composition is osteogenic and osteoinductive. The bone building materials may be contained in or coated onto the surface of the composition.

"Calcium containing" materials include hydroxyapatite, monobasic, dibasic and tribasic calcium phosphates, calcium aluminates, calcium containing ceramics, porous calcium containing ceramic particles and amorphous calcium phosphate.

As used herein, a "nutrient factor" is a compound or series of compounds used to sustain metabolic activities or used to promote normal physiologic function or optimal health. Nutrient factors include vitamins, hormones, individual or combinations of amino acids, carbohydrates or derivatives thereof, fats or derivatives thereof, alcohols or derivatives thereof, inorganic salts and trace elements.

As used herein, a "Bone Morphogenic Protein" is any of the zinc metalloendopeptidase enzymes that are involved in induction of bone and cartilage formation. Bone Morphogenic Proteins include Bone Morphogenic Protein-2 (BMP-2), Bone Morpbogenic Protein-2a (BMP-2a), Bone Morphogenic Protein-4 (BMP-4), Bone Morphogenic Protein-5 (BMP-5), Bone Morphogenic Protein-6 (BMP-6), Bone Morphogenic Protein-7 (BMP-7) and Bone Morphogenic Protein-8 (BMP-8).

As used herein, a "growth factor" is a substance that is operable to increase the size of a living being or any of its parts or to stimulate cell growth. Growth factors include Transforming Growth Factor-beta (TGF-β), Transforming Growth Factor-alpha (TGF-α), Epidermal Growth Factor (EGF), Insulin-like Growth Factor-I or II, Interleuldn-I, Interferon, Tumor Necrosis Factor, Fibroblast Growth Factor (FGF), Platelet Derived Growth Factor (PDGF) and Nerve Growth Factor (NGF).

As used herein, an "anti-inflammatory" is an agent that reduces inflammation without directly antagonizing the causative agent. "Anti-inflammatories" include steroidal and non-steroidal anti-inflammatory agents.

As used herein, a "blood product" is a product, any component of which is derived from blood. Blood products include whole blood and blood fractions, such as plasma, blood cells, blood factors, blood related proteins, unspecialized cells such as stem cells (including adipose derived stem cells), or specialized cells, e.g., types of leukocytes such as lymphocytes and dendritic cells.

Other suitable materials may include inorganic materials, metals, such as mesh titanium or titanium alloy, amino acids, gelatin, collagen, naturally occurring or synthetic therapeutic drugs, proteins and enzymes.

The bone repairing composition is formed into a shape. As used herein, "formed" refers to a rigid object having fixed dimensions and specific volume due to the cohesion of its components. The formed shape may be a block, disc, patch, ring, cylinder or be site-specific preformed to fit the injury site.

### Methods of Preparation and Use of Formed Bone Composition

The present invention provides methods of making a formed composition and methods of augmenting bone at a site in need of augmentation. Such methods include those for making a formed composition for application to a bone surface of a human or animal subject, said methods comprising:
(a) mixing denatured demineralized bone and water;
(b) heating the mixture of denatured demineralized bone and water to form a carrier;
(c) mixing the carrier with a bone material to form a moldable composition;
(d) molding the moldable composition; and
(e) lyophilizing the moldable composition to produce a rigid formed composition having a shape suitable for administration to the bone

### Preparing a Formed Composition

### Preparing the Bone

Bone is collected from a donor source and may include the entire bone or bone fragments from cancellous or cortical bone. In a preferred embodiment, the subject is of the same species as the donor. For example, all of the bone used to prepare a composition for a human patient may be sourced from a single human cadaveric donor. Any adherent tissues may be removed from the bone by standard bone cleaning protocol.

In various embodiments, the bone is milled into particles ranging from 700 microns to 2000 microns. As used herein, the term "milled" and conjugations thereof, refers to shaping a tissue to the desired size by crushing, chopping, cutting, shaving, grinding or pulverizing. In embodiments where several sizes of bone are be used, it is understood that the milling process may be repeated and the respective bone portions may be reserved and assigned accordingly. Commercially available milling and sieving devices may be used or bone may be purchased in the form of an allograft matrix in the desired particle size or sizes.

Milled bone may be defatted by soaking or washing the bone in ethanol because the high polarity of ethanol solubizes the less polar lipids. A preferred ethanol solution is at least 60% ethanol, volume to volume, in deionized/distilled water. A more preferred ethanol solution is 100% ethanol. The ethanol bath also disinfects the bone by killing vegetative microorganisms and viruses. A further antiseptic step may include treatment of the milled bone with a hydrogen peroxide solution.

In embodiments containing natural bone chips, a portion of the milled bone may be set aside before demineralizing of the other components.

### Preparing the Bone Material

To prepare the bone material, milled bone is demineralized using an acidification or chelating process. Acids used include inorganic acids such as hydrochloric acid or organic acids such as peracetic acid. Chelating agents include disodium ethylenediaminetetraacetic acid (Na₂EDTA).

The time required to demineralize the bone may vary depending on the concentration of acid or chelating agent used, the displacement or flow of the solution and the desired final concentration of calcium in the bone. For example, in an embodiment using hydrochloric acid, at an acid concentration of 0.1 to 2.0 N, the bones may be soaked in the acid bath for up to 24 hours. The calcium or mineral concentration in the milled bone may be monitored by measuring the pH of the acid solution using a calcium specific electrode or a standard pH meter. In a preferred embodiment, the acid wash or soak ceases when the calcium concentration of the bone is less than 1%.

After demineralization, the pH of the bone is adjusted by removing the acid with a deionized/distilled water wash until the pH of the bone approximates that of the water. It is not outside of the scope of embodiments of this invention to expedite the neutralization of the bone using an ionic strength adjuster such as a biocompatible buffer solution.

Bone for the bone material may then be lyophilized to a moisture level of less than 6% using standard drying techniques including, but not limited to, freeze drying, vacuum drying and evaporation.

### Preparing the Carrier Component

To prepare the carrier component, the milled bone is demineralized according to the procedure set forth above. The demineralized bone is then added to an aqueous component such as water or a saline solution. The demineralized bone may be in a wet, moist or dry state or a combination of states. Each 5 to 25 grams of demineralized bone requires the addition of about 100 grams of water or a saline solution. It is understood that adjustments may be made to these ratios depending on the bone size and bone state (chips, powder, fragments, etc.).

The carrier is then heat treated. Suitable heat treatments incorporate boiling, steaming or the use of an oven. Preferably, the carrier is autoclaved at a temperature of from 100°C to 150°C, at a pressure, of from 68.69 kPa (10 psi) to 137.9 kPa (20 psi), for a period of a 0 minutes to 2 hours. In a preferred embodiment, the mix is autoclaved at 121°C under a pressure of 15 psi for 60 minutes. The duration of autoclaving may be adjuster depending upon the amount of demineralized bone and the amount and type of liquid used.

### Preparing the Moldable Material

The carrier component and bone material component are combined to form a paste or moldable material. This mixing may be achieved when the carrier component is mostly in the liquid state or when it has formed a gelatinous mass such as that achieved by cooling. The mixing may be performed in a separate container or it may be performed in the mold, as detailed later herein.

Embodiments of this invention consist of about 100 grams of the carrier component mixed with about 25 to about 40 grams of the bone material component. In a preferred embodiment, about 100 grams of the carrier component is mixed with about 27 to 35 grams of the bone material component. Depending on the formulation used, the carrier component comprises from 72% to 80% of the paste weight and the bone material component comprises from 20% to 28% of the paste weight.

In various embodiments containing bone chips, the bone chips comprise 10% of the bone material weight or 2% of the total paste weight. For example, in preferred embodiments where 28 grams of the bone material component is used, 2.8 grams of natural bone chips are added to the paste. The bone chips may be added during or after mixing of the carrier component and bone material component. Bone building materials, such as those described herein, may also be added during or after the paste preparation step. The timing of addition is important because the bone building properties of the material may be compromised if the material is added before the demineralization step. For example, the bone enhancing qualities of supplemental calcium phosphate would be futile because it would wash away during the acidification or chelating process. Nonetheless, it is possible to add other biologically active agents in the formulation at this stage. These biological agents include, for example, antibiotics and growth factors.

### Preparing the Formed Composition

This paste is then "cast" into the formed shape. As used herein, the term "cast" relates to the process of making impressions or of shaping in a mold. The casts may be formed by placing the moldable material into sterilized and possibly disposable molds. The paste may be placed into the mold by spreading with a spatula type device or dispensing with a syringe, for example.

In various embodiments, the filled mold may be placed inside of a sterilized dual chamber package. Packaging is preferably durable, flexible, has barrier resistance to moisture, chemicals, grease and bacteria, maintains its integrity upon exposure to low temperatures and is easy to handle in a medical or clinical setting. Suitable packaging materials may include materials selected from the group consisting of thermoplastic films, polyester films, para-aramid fibers, polyethylene fibers, and combinations thereof. In a preferred embodiment, the inner packaging includes a polyester film, such as Molars and a polyethylene fiber, such as Tyvek® (both DuPont, Wilmington, Deleware, USA) and the outer compartment is a moisture resistant foil bag made of aluminum and transparent plastic with a Tyvek® Header pouch. Moisture may be drawn from the filled Tyvek Mylar® aluminum/plastic chamber by lyophilizing, vacuum drying, air drying, temperature flux drying, molecular sieve drying and other suitable drying techniques. preferably, moisture is removed by lyophilizing until the moisture content decreases to about 6% of the cast weight. In a preferred embodiment, the moisture level is less than 6%.

In an embodiment where the bone building material is loaded after the paste preparation step, the mold may be lined with the bone building material or biologically active ingredients which coat the outer surface of the composition. The mold may also incorporate structural features such as ridges, corrugation or other surface indentations to impart structural stability and rigidity.

In various embodiments where the paste may be placed into a cast using a syringe, a system may be used which incorporates the mold and places it in communication with a syringe. Suitable devices are discussed later herein.

The formed composition may have a generic or site specific shape. Generic formed compositions include sheets, patches, rings, cubes, cylinders or discs to be formed to an injury site during surgery. In embodiments where the formed shape is a patch or sheet, the rigidity of the composition may be altered. A sheet material which is more pliable or less pliable may be accomplished by changing the sheet thickness or adding ridges or corrugation, for example.

A site specific formed composition may have the dimensions of the void to be filled and does not require additional manipulation in the operating room. The dimensions may be acquired using an x-ray of the site of the defect as a reference for size and shape. The x-ray may be scaled to the appropriate dimensions for the cast. Depending on the quantity and type of bone defect repairs required, a plurality of generic and site specific formed compositions may be used during the surgery.

### Methods of Augmenting a Bone Site

Embodiments of this invention may be used to repair bone defects. As used herein, "bone defects" or "injury sites", and variants thereof, refer to bone imperfections caused by birth defect, trauma, disease, decay or surgical intervention, and the desired repair may be for cosmetic or therapeutic reasons.

Embodiments of the bone repairing composition may be used to correct bone defects in orthopedic, neurosurgical plastic or reconstructive surgery, in periodontal procedures, and in endodontic procedures. Examples include repair of simple and compound fractures and non-unions, external and internal fixations, joint reconstruction such as arthrodesis, general arthroplasty, cup arthroplasty of the hip, femoral and humeral head replacement, femoral head surface replacement and total joint replacement, repairs of the vertebral column including spinal fusion and internal fixation, tumor surgery, e.g. deficit filing, discectomy, laminectomy, excision of spinal cord tumors, anterial cervical and thoracic operations, repair of spinal injuries, scoliosis, lordosis and kyphosis treatments, intermaxillary fixation of fractures, mentoplasty, temporomandibular joint replacement, alveolar ridge augmentation and reconstruction, inlay bone grafts, implant placement and revision, sinus lifts, etc. The standard surgical and dental procedures are suitable for use with the various methods. (See, e.g., U.S. Patent Nos. 6,180,606, Chen, et al., issued January 30, 2001 and 5,507,813, Dowd, et al., issued April 16, 1996.)

An aqueous solution, preferably containing water, is added to the dried bone repairing composition and the composition may be placed into the site or defect. In one embodiment, adding water to the dried bone may be achieved by adding blood to the composition. Hydration blood includes, but is not limited to, whole blood and blood components such as, red blood cells and components, white blood cells and components, plasma, plasma fractions, plasma serum, platelet concentrate, blood proteins, thrombin, and coagulation factors.

In embodiments where the formed composition is in sheet or patch form, the surgeon may simply place a single patch or several patches in the defect and shape it appropriately by hand or with a surgical tool. When the device is site specific preformed, the surgeon may match the contour of the composition with the contour of the injury and inserts the composition into the void. Any combination of site specific or generic patches may be used to fill a defect.

The formed composition may reconstitute or rehydrate while in the defect site. Ambient fluids such as blood are absorbed after a few minutes. Extra corpus fluids, including but not limited to, saline, water or a balanced salt solution (140 mm NaCl, 5.4 mm KCl, pH 7.6) are used to expedite the hydration. In an alternative embodiment, the device may be reconstituted away from the defect site using the subject's blood or extra corpus fluids. As described later herein, various hydration apparatus may also be used to facilitate hydration of the formed composition before augmenting the bone site.

The compositions may be made pliable to soften the device, allowing for easy manipulation and fit into the defect site. Suitable methods include application of heat or hydration by the direct application of warm aqueous based solutions to the formed composition. In various embodiments, a heating element may be used to transfer thermal energy to the formed composition. Suitable heating elements may use electrical, mechanical or chemical means to generate the thermal energy. For example, a heat pack may include a self-contained and user activated exothermic chemical means to generate heat and the pack may be disposed adjacent to or enclose a receptacle containing the formed composition. Upon initiating the exothermic reaction, heat is transferred through the heat pack and to the formed composition. Exemplary heating devices are disclosed in U.S. Patent No. 5,263,991, Wiley, et al, issued November 23, 1993, incorporated by reference. It is understood that the appropriate temperature and timing of the heat application depends on the dimensions, quantity and contents of the formed composition(s) and the selected heating techniques.

### Examples

### Example 1

Cancellous bone is harvested and adherent tissue are removed. The bone is milled into particles of 1500 microns and 810 microns. The bone is soaked in a solution of 100% ethanol, volume to volume, in deionized/distilled water to remove fat and kill microorganisms. The larger (1500 microns) bone particles are set aside. The smaller (810 microns) bone particles are placed in a 0.5 N hydrochloric acid bath and soaked overnight. The calcium ion concentration of the bone particles is monitored by measuring the ion concentration of the acid solution with a calcium specific electrode. The calcium concentration reaches 1% and the bone is removed from the acid bath. The de-calcified bone is washed with deionized/distilled water until the runoff rinse solution reaches a neutral pH level. To prepare a bone repairing composition, 25 grams of the demineralized bone (1% calcium, 810 microns) is mixed with 100 grams of a saline solution. The mixture is autoclaved at 121°C under a pressure of 15 psi for 60 minutes to form the gel carrier. The carrier is mixed with 28 grams of demineralized bone (1% calcium, 810 microns) and a paste is formed. Additionally, 2.8 grams of the reserved natural bone chips (1500 microns) are added to the paste. The paste is spread into a square shaped mold using a spatula. The mold and paste are placed inside of a sterilized Tyvck®Mylar® dual chamber package. Moisture is withdrawn from the package by lyophilization and removed until the moisture content in the paste is less than about 6% of the cast weight. The freeze dried formed bone composition is a square patch measuring 4 cm x 4 cm and having a thickness of 0.3 cm.

### Example 2

A bone repairing composition is prepared according to the method described in Example 1. The carrier comprises 100 grams of saline and 20 grams of demineralized bone. The paste is formed by adding 30 grams of bone material to the carrier. The paste is placed into a tubular shaped mold which is lined with platelet concentrate to coat outer surfaces of the composition. The final dried composition is a cylinder having a diameter of 1.25 cm and a base height of 2.5 cm.

### Example 3

A bone repairing composition is prepared according to the method of Example 1. The carrier comprises 100 grams of saline and 15 grams of demineralized bone. The paste is formed by adding 32.85 g of bone material to the carrier. Additionally, 3.285 grams of reserved natural bone chips are added. The composition is formed into a rectangular patch, and lyophilized. The pre-lyophilization mixture has the following weight percentages: 9.92% of the carrier, 21.74% of the bone material component, 66.17% water and 2.17% natural bone chips. After lyophilization, the final formed composition has the following weight percentages: 29.33% carrier component, 64.24% bone material component and 6.41% natural bone chips.

### Example 4

A patch for use in craniofacial surgery is prepared according to the method of Example 1. The carrier component comprises 100 grams of saline and 25 grams of demineralized bones The paste is formed by adding 35.714g of demineralized bone to the carrier. Additionally, 3.57 grams of natural bone chips are added. The patch has the following weight percentages: 38.89% carrier component, 55.56% bone material component and 5.55% natural bone chips. The patch is a circle having a diameter of 9.5 cm and a thickness of 0.5 em. The patch is hydrated while in the injury site using the subject's blood as the source of water.

The patch is heated using the LactoSorb® Heat Pack (Biomet, Inc.; Warsaw, Indiana, USA) for 1 minute prior to application into the injury site. The patch is malleable thereby facilitating anatomical contouring into the injury site. The patch is hydrated while in the injury site using the subject's blood as the source of water.

### Example 5

Spinal surgery fusion is enhanced by using a combination of patch and cylindrical shaped formed compositions according to Examples 1 and 2. In this example, the formed compositions are coated with calcium triphosphate and Bone Morphogenic Protein-2 prior to implantation.

### Example_ 6

An x-ray is taken of a subject's fractured hip. The x-ray dimensions are scaled to match the subject's height/size and a site specific cast composition according to Example 1 is prepared. The composition is implanted at the site of the fracture.

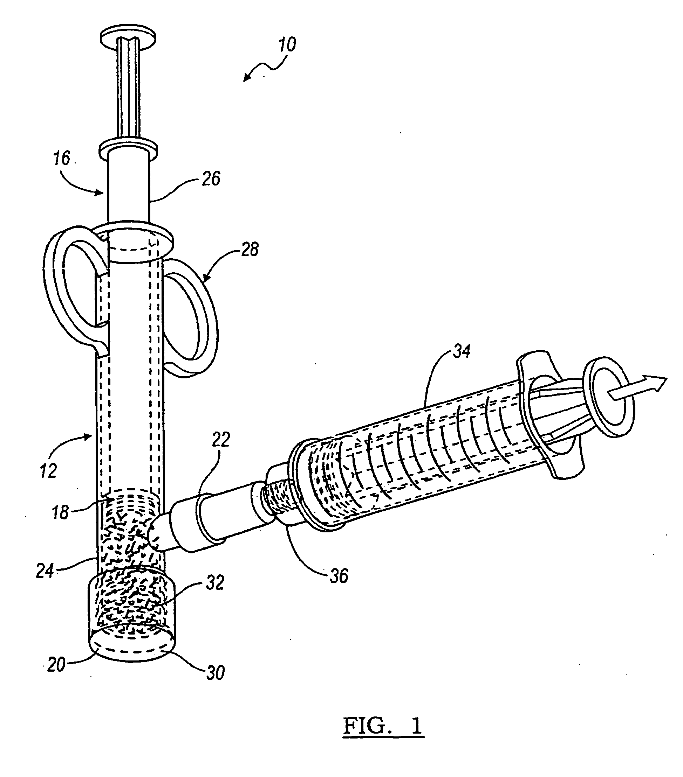

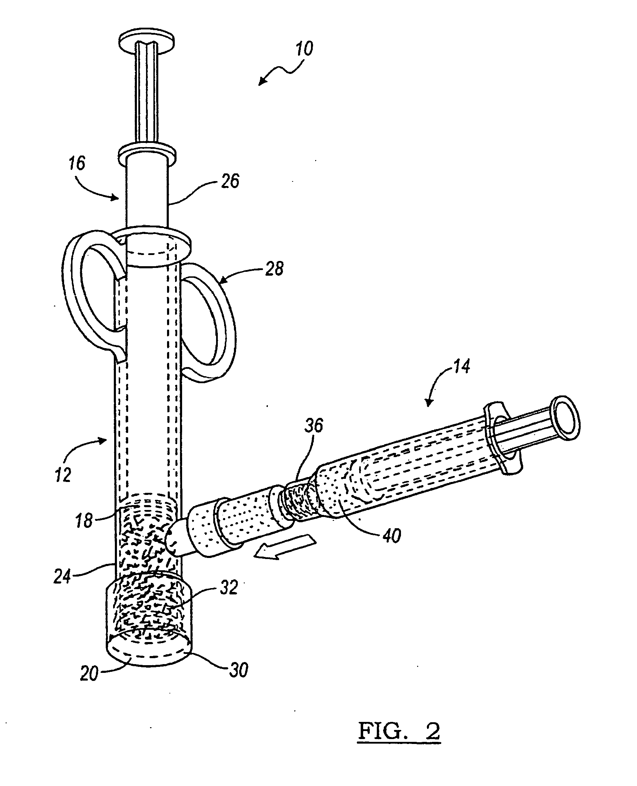

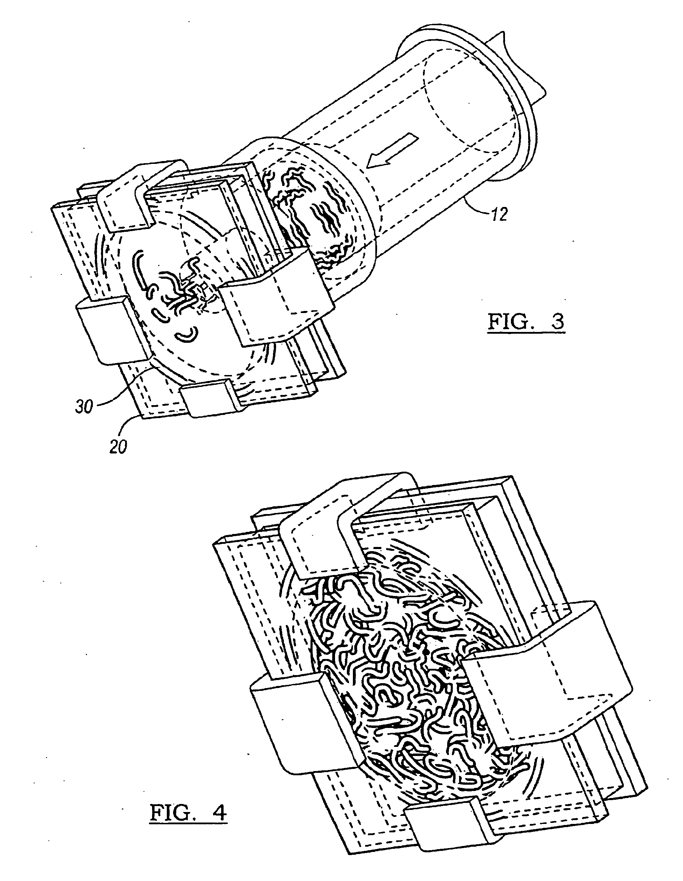

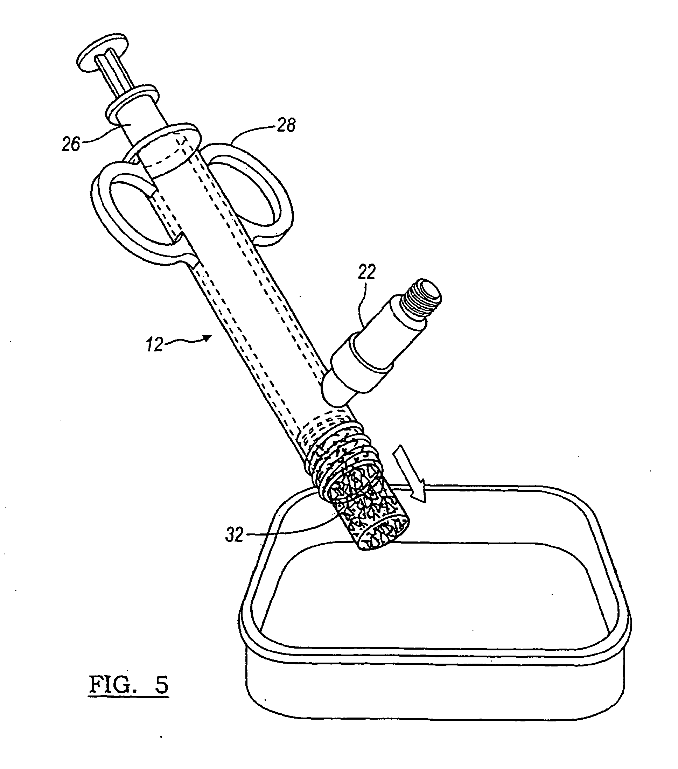

## Claims

1. A rigid formed composition for application to a bone surface of a human or animal subject, comprising:
(a) a bone material; and
(b) a carrier comprising denatured demineralized bone;
wherein the composition is formed into a rigid shape suitable for administration to the bone, wherein the carrier is made by a process comprising:
(a) mixing demineralized bone with water; and
(b) heating the mixture of demineralized bone and water under pressure, wherein the heating comprises autoclaving the mixture.

2. A rigid formed composition according to Claim 1, wherein the bone material is selected from the group consisting of: bone powder, bone chips, bone shavings, and mixtures thereof

3. A rigid formed composition according to Claim 2, wherein the bone material comprises demineralized bone powder.

4. A rigid formed composition according to Claim 3, wherein the demineralized bone consists essentially of demineralized bone powder.

5. A rigid formed composition according to Claim 3, wherein the demineralized bone powder has a particle size of less than 1000 microns.

6. A rigid formed composition according to Claim 5, wherein the demineralized bone powder has a particle size of less than 850 microns.

7. A rigid formed composition according to Claim 3, wherein the demineralized bone additionally comprises demineralized bone chips.

8. A rigid formed composition according to Claim 7, wherein the large dimension of the demineralized bone chips is from 750 to 2000 microns.

9. A rigid formed composition according to Claim 8, wherein the large dimension is from 750 to 1500 microns.

10. A rigid formed composition according to Claim 1, comprising from 10% to 40% of the bone material.

11. A rigid formed composition according to Claim 10, comprising from 20% to 30% of the bone material.

12. A rigid formed composition according to Claim 1, wherein the carrier comprises from 0.2% to 40% (by weight of carrier) of the denatured demineralized bone.

13. A rigid formed composition according to Claim 12, wherein the carrier comprises from 0.5% to 25% of the denatured demineralized bone.

14. A rigid formed composition according to Claim 13, wherein the carrier comprises from 10% to 20% of the denatured demineralized bone.

15. A rigid formed composition according to Claim 1, additionally comprising a bone building material.

16. A rigid formed composition according to Claim 15, wherein the bone building material is selected from the group consisting of calcium-containing materials, nutrient factors, bone morphogenic proteins, growth factors, antimicrobials, anti-inflammatory agents, blood products, and mixtures thereof.

17. A rigid formed composition according to Claim 16, wherein the bone building material comprises a calcium-containing ceramic material.

18. A rigid formed composition according to Claim 15, wherein the bone building material comprises a bone morphogenic protein, a growth factor, or mixtures thereof.

19. A rigid formed composition according to Claim 1, additionally comprising a blood product.

20. A rigid formed composition according to Claim 1, wherein the shape is a sheet, a patch, a block, a ring, a disc, a cylinder, or a site-specific pre-form.

21. A rigid formed composition according to Claim 20, wherein the shape is a patch.

22. A rigid formed composition according to Claim 1, wherein the bone material and the denatured demineralized bone are autologous with the subject.

23. A method for making a rigid formed composition for application to a bone surface of a human or animal subject, the method comprising:
(a) mixing denatured demineralized bone and water;
(b) heating the mixture of denatured demineralized bone and water to form a carrier;
(c) mixing the carrier with a bone material to form a moldable composition;
(d) molding the moldable composition; and
(e) lyophilizing the moldable composition to produce a rigid formed composition having a shape suitable for administration to the bone.

24. A method according to Claim 23, wherein said heating comprises autoclaving said mixture.

25. A method according to Claim 24, wherein said autoclaving is conducted at a temperature of from 100°C to 15°C, at a pressure of from 68.96 kPa (10psi) to 137.79 kPa (20 psi), for from 0 min to 2 hours.

26. A method according to Claim 23, further comprising packaging said composition after lyophilizing in a substantially air-impermeable package.

27. A method according to Claim 26, wherein said package comprises a material selected from the group consisting of thermoplastic films, polyester films, para-aramid fibers, polyethylene fibers, and combinations thereof.

28. A rigid product obtainable by the method of Claims 23 to 27.

29. Use of a rigid formed composition according to Claims 1 to 22 for the preparation of a bone repairing composition useful in repairing osseous defects by a method of augmenting bone at a site in need thereof in a human or animal subject, comprising:
(a) adding water to a dried composition comprising:
(i) a bone material; and
(ii) a carrier comprising denatured demineralized bone; wherein said composition is formed into a rigid shape suitable for administration to said bone; and
(b) applying said rigid formed composition to said site.

30. A use according to Claim 29, wherein said adding of water comprises adding blood to said composition.

31. A use according to Claim 29, further comprising shaping said rigid formed composition after said applying, to conform said composition to features of said site.

32. A use according to Claim 29, wherein said bone material comprises demineralized bone powder.

33. A use accruing to Claim 32, wherein said bone material additionally comprises demineralized bone chips.

34. A use according to Claim 29, wherein said rigid formed composition additionally comprises a bone building material.

35. A use according to Claim 34, wherein said rigid formed composition additionally comprises a calcium-containing ceramic material.

36. A use according to Claim 34, wherein said bone building material comprises a bone morphogenic protein, a growth factor, or mixtures thereof.

37. A use according to Claim 29, wherein said shape is a patch, a sheet, a block, a ring, a disc, or a site-specific pre-form.

38. A use according to Claim 29, wherein said bone material and said denatured demineralized bone are from the same subject.

39. A use according to Claim 29, further comprising heating said rigid formed bone composition.

40. A use according to Claim 39, wherein said heating comprises using a heat pack.

## Patentansprüche

1. Steife, geformte Zusammensetzung zur Auftragung auf eine Knochenoberfläche eines menschlichen oder tierischen Subjekts, umfassend:
(a) ein Knochenmaterial und
(b) einen Träger, der denaturierten, demineralisierten Knochen umfasst;
wobei die Zusammensetzung zu einer steifen Form geformt ist, die für eine Verabreichung an den Knochen geeignet ist, wobei der Träger durch ein Verfahren hergestellt wird, das umfasst:
(a) Mischen von demineralisiertem Knochen mit Wasser und
(b) Erwärmen des Gemisches aus demineralisiertem Knochen und Wasser unter Druck, wobei das Erwärmen Autoklavieren des Gemisches umfasst.

2. Steife, geformte Zusammensetzung gemäß Anspruch 1, wobei das Knochenmaterial aus der Gruppe, bestehend aus Knochenpulver, Knochenschnitzeln, Knochenspänen und Gemischen davon, ausgewählt ist.

3. Steife, geformte Zusammensetzung gemäß Anspruch 2, wobei das Knochenmaterial demineralisiertes Knochenpulver umfasst.

4. Steife, geformte Zusammensetzung gemäß Anspruch 3, wobei der demineralisierte Knochen im Wesentlichen aus demineralisertem Knochenpulver besteht.

5. Steife, geformte Zusammensetzung gemäß Anspruch 3, wobei das demineralisierte Knochenpulver eine Partikelgröße von weniger als 1000 Mikrometer hat.

6. Steife, geformte Zusammensetzung gemäß Anspruch 5, wobei das demineralisierte Knochenpulver eine Partikelgröße von weniger als 850 Mikrometer hat.

7. Steife, geformte Zusammensetzung gemäß Anspruch 3, wobei der demineralisierte Knochen zusätzlich demineralisierte Knochenschnitzel umfasst.

8. Steife, geformte Zusammensetzung gemäß Anspruch 7, wobei die große Abmessung der demineralisierten Knochenschnitzel 750 bis 2000 Mikrometer ist.

9. Steife, geformte Zusammensetzung gemäß Anspruch 8, wobei die große Abmessung 750 bis 1500 Mikrometer ist.

10. Steife, geformte Zusammensetzung gemäß Anspruch 1, die 10% bis 40% Knochenmaterial umfasst.

11. Steife, geformte Zusammensetzung gemäß Anspruch 10, die 20 bis 30% Knochenmaterial umfasst.

12. Steife, geformte Zusammensetzung gemäß Anspruch 1, wobei der Träger 0,2% bis 40% (bezogen auf das Gewicht des Trägers) denaturierten, demineralisierten Knochen umfasst.

13. Steife, geformte Zusammensetzung gemäß Anspruch 12, wobei der Träger 0,5% bis 25% denaturierten, demineralisierten Knochen umfasst.

14. Steife, geformte Zusammensetzung gemäß Anspruch 13, wobei der Träger 10% bis 20% denaturierten, demineralisierten Knochen umfasst.

15. Steife, geformte Zusammensetzung gemäß Anspruch 1, die außerdem ein knochenbildendes Material umfasst.

16. Steife, geformte Zusammensetzung gemäß Anspruch 15, wobei das knochenbildende Material ausgewählt ist aus der Gruppe, bestehend aus: Calcium-enthaltenden Materialien, Nährstofffaktoren, morphogenen Knochenproteinen, Wachstumsfaktoren, antimikrobiellen Mitteln, antiinflammatorischen Mitteln, Blutprodukten und Gemischen davon.

17. Steife, geformte Zusammensetzung gemäß Anspruch 16, wobei das knochenbildende Material ein Calcium-enthaltendes Keramikmaterial umfasst.

18. Steife, geformte Zusammensetzung gemäß Anspruch 16, wobei das knochenbildende Material ein morphogenes Knochenprotein, einen Wachstumsfaktor oder Gemische davon umfasst.

19. Steife, geformte Zusammensetzung gemäß Anspruch 1, die zusätzlich ein Blutprodukt umfasst.

20. Steife, geformte Zusammensetzung gemäß Anspruch 1, wobei die Form eine Folie, ein Pflaster, ein Block, ein Ring, eine Scheibe, ein Zylinder oder eine stellenspezifische Vorform ist.

21. Steife, geformte Zusammensetzung gemäß Anspruch 20, wobei die Form ein Pflaster ist.

22. Steife, geformte Zusammensetzung gemäß Anspruch 1, wobei das Knochenmaterial und der denaturierte, demineralisierte Knochen mit dem Subjekt autolog sind.

23. Verfahren zur Herstellung einer steifen, geformten Zusammensetzung zur Auftragung auf eine Knochenoberfläche eines menschlichen oder tierischen Subjekts, wobei das Verfahren umfasst:
(a) Mischen von denaturiertem, demineralisiertem Knochen und Wasser;
(b) Erwärmen des Gemisches aus denaturiertem, demineralisiertem Knochen und Wasser unter Bildung eines Trägers;
(c) Mischen des Trägers mit einem Knochenmaterial unter Bildung einer formbaren Zusammensetzung;
(d) Formen der formbaren Zusammensetzung und
(e) Lyophilisieren der formbaren Zusammensetzung unter Herstellung einer steifen, geformten Zusammensetzung, die eine Form hat, die zur Verabreichung an den Knochen geeignet ist.

24. Verfahren gemäß Anspruch 23, wobei das Erwärmen Autoklavieren des Gemisches umfasst.

25. Verfahren gemäß Anspruch 24, wobei das Autoklavieren bei einer Temperatur von 100°C bis 150°C, bei einem Druck von 68,96 kPa (10 psi) bis 137,79 kPa (20 psi), für 0 min bis 2 Stunden durchgeführt wird.

26. Verfahren gemäß Anspruch 23, das außerdem das Verpacken der Zusammensetzung nach Lyophilisierung in einer im Wesentlichen luftundurchlässigen Verpackung umfasst.

27. Verfahren gemäß Anspruch 26, wobei die Verpackung ein Material umfasst, das ausgewählt ist aus der Gruppe, bestehend aus thermoplastischen Filmen, Polyesterfilmen, Para-Aramid-Fasern, Polyethylenfasern und Kombinationen davon.

28. Steifes Produkt, erhältlich durch das Verfahren gemäß den Ansprüchen 23 bis 27.

29. Verwendung einer steifen, geformten Zusammensetzung gemäß den Ansprüchen 1 bis 22 zur Herstellung einer Knochenreparaturzusammensetzung, die bei der Reparatur von Knochendefekten durch ein Verfahren der Verstärkung des Knochens an einer Stelle, die Bedarf dafür hat, bei einem menschlichen oder tierischen Subjekt verwendbar ist, umfassend:
(a) Zugeben von Wasser zu einer getrockneten Zusammensetzung, umfassend:
(i) ein Knochenmaterial und
(ii) einen Träger, der denaturierten, demineralisierten Knochen umfasst, wobei die Zusammensetzung in eine steife Form geformt ist, die zur Verabreichung an den Knochen geeignet ist, und
(b) Auftragen der steifen, geformten Zusammensetzung auf die Stelle.

30. Verwendung gemäß Anspruch 29, wobei das Zusetzen von Wasser Zusetzen von Blut zu der Zusammensetzung umfasst.

31. Verwendung gemäß Anspruch 29, die außerdem Formen der steifen, geformten Zusammensetzung nach dem Auftragen umfasst, um die Zusammensetzung an Merkmale der Stelle anzupassen.

32. Verwendung gemäß Anspruch 29, wobei das Knochenmaterial demineralisiertes Knochenpulver umfasst.

33. Verwendung gemäß Anspruch 32, wobei das Knochenmaterial außerdem demineralisierte Knochenschnitzel umfasst.

34. Verwendung gemäß Anspruch 29, wobei die steife, geformte Zusammensetzung zusätzlich ein knochenbildendes Material umfasst.

35. Verwendung gemäß Anspruch 34, wobei die steife, geformte Zusammensetzung zusätzlich ein Calcium-enthaltendes Keramikmaterial umfasst.

36. Verwendung gemäß Anspruch 34, wobei das knochenbildende Material ein morphogenes Knochenprotein, einen Wachstumsfaktor oder Gemische davon umfasst.

37. Verwendung gemäß Anspruch 29, wobei die Form ein Pflaster, eine Folie, ein Block, ein Ring, eine Scheibe oder eine stellenspezifische Vorform ist.

38. Verwendung gemäß Anspruch 29, wobei das Knochenmaterial und der denaturierte, demineralisierte Knochen von demselben Subjekt sind.

39. Verwendung gemäß Anspruch 29, die außerdem Erwärmen der steifen, geformten Knochenzusammensetzung umfasst.

40. Verwendung gemäß Anspruch 39, wobei das Erwärmen die Verwendung einer Wärmepackung umfasst.

## Revendications

1. Composition formée rigide destinée à une application sur la surface d'un os d'un sujet humain ou animal, comprenant :
(a) un matériau osseux ; et
(b) un support comprenant de l'os déminéralisé dénaturé ;
où la composition est mise sous une forme rigide appropriée pour une administration à l'os, où le support est fabriqué par un procédé comprenant :
(a) le mélange d'os déminéralisé avec de l'os ; et
(b) le chauffage du mélange d'os déminéralisé et d'eau sous pression,
où le chauffage comprend l'autoclavage du mélange.

2. Composition formée rigide selon la revendication 1, dans laquelle le matériau osseux est choisi dans le groupe constitué de : poudre d'os, d'éclats d'os, de copeaux d'os et de mélanges de ceux-ci.

3. Composition formée rigide selon la revendication 2, dans laquelle le matériau osseux comprend de la poudre d'os déminéralisé.

4. Composition formée rigide selon la revendication 3, dans laquelle l'os déminéralisé est constitué essentiellement de poudre d'os déminéralisé.

5. Composition formée rigide selon la revendication 3, dans laquelle la poudre d'os déminéralisé présente une taille de particule inférieure à 1000 microns.

6. Composition formée rigide selon la revendication 5, dans laquelle la poudre d'os déminéralisé présente une taille de particule inférieure à 850 microns.

7. Composition formée rigide selon la revendication 3, dans laquelle l'os déminéralisé comprend des éclats d'os déminéralisé.

8. Composition formée rigide selon la revendication 7, dans laquelle la grande dimension des éclats d'os déminéralisé est de 750 à 2000 microns.

9. Composition formée rigide selon la revendication 8, dans laquelle la grande dimension est de 750 à 1500 microns.

10. Composition formée rigide selon la revendication 1, comprenant de 10 % à 40 % du matériau osseux.

11. Composition formée rigide selon la revendication 10, comprenant de 20 % à 30 % du matériau osseux.

12. Composition formée rigide selon la revendication 1, dans laquelle le support comprend de 0,2 % à 40 % (en poids de support) de l'os déminéralisé dénaturé.

13. Composition formée rigide selon la revendication 12, dans laquelle le support comprend de 0,5 % à 25 % de l'os déminéralisé dénaturé.

14. Composition formée rigide selon la revendication 13, dans laquelle le support comprend de 10 % à 20 % de l'os déminéralisé dénaturé.

15. Composition formée rigide selon la revendication 1, comprenant en outre un matériau de construction d'os.

16. Composition formée rigide selon la revendication 15, dans laquelle le matériau de construction d'os est choisi dans le groupe constitué de : matériaux contenant du calcium, facteurs nutritifs, protéines morphogéniques osseuses, facteurs de croissance, antimicrobiens, agents anti-inflammatoires, produits sanguins et mélanges de ceux-ci.

17. Composition formée rigide selon la revendication 16, dans laquelle le matériau de construction d'os comprend un matériau céramique contenant du calcium.

18. Composition formée rigide selon la revendication 16, dans laquelle le matériau de construction d'os comprend une protéine morphogénique osseuse, un facteur de croissance ou des mélanges de ceux-ci.

19. Composition formée rigide selon la revendication 1, comprenant en outre un produit sanguin.

20. Composition formée rigide selon la revendication 1, où la forme est un feuillet, un patch, un bloc, un anneau, un disque, un cylindre ou une préforme spécifique du site.

21. Composition formée rigide selon la revendication 20, où la forme est un patch.

22. Composition formée rigide selon la revendication 1, dans laquelle le matériau osseux et l'os déminéralisé dénaturé sont autologues avec le sujet.

23. Procédé de fabrication d'une composition formée rigide destinée à une application sur la surface d'un os d'un sujet humain ou animal, le procédé comprenant :
(a) le mélange d'os déminéralisé dénaturé et d'eau ;
(b) le chauffage du mélange d'os déminéralisé dénaturé et d'eau pour former un support ;
(c) le mélange du support avec un matériau osseux pour former une composition modelable ;
(d) le modelage de la composition modelable ; et
(e) la lyophilisation de la composition modelable pour produire une composition formée rigide ayant une forme appropriée pour une administration à l'os.

24. Procédé selon la revendication 23, dans lequel ledit chauffage comprend l'autoclavage dudit mélange.

25. Procédé selon la revendication 24, dans lequel ledit autoclavage est conduit à une température de 100 °C à 150 °C, à une pression de 68,96 kPa (10 psi) à 137,79 kPa (20 psi), pendant 0 min à 2 heures.

26. Procédé selon la revendication 23, comprenant en outre le conditionnement de ladite composition après lyophilisation dans un conditionnement pratiquement imperméable à l'air.

27. Procédé selon la revendication 26, dans lequel ledit conditionnement comprend un matériau choisi dans le groupe constitué de films thermoplastiques, de films de polyester, de fibres de para-aramide, de fibres de polyéthylène et de combinaisons de ceux-ci.

28. Produit rigide pouvant être obtenu par le procédé selon les revendications 23 à 27.

29. Utilisation d'une composition formée rigide selon les revendications 1 à 22 pour la préparation d'une composition de réparation osseuse utile dans la réparation d'anomalies osseuses par un procédé consistant à augmenter l'os au niveau d'un site en ayant besoin chez un sujet humain ou animal, comprenant :
(a) l'addition d'eau à une composition séchée comprenant :
(i) un matériau osseux ; et
(ii) un support comprenant de l'os déminéralisé dénaturé ; où ladite composition est mise sous une forme rigide appropriée pour une administration au dit os ; et
(b) l'application de ladite composition formée rigide au dit site.

30. Utilisation selon la revendication 29, où ladite addition d'eau comprend l'addition de sang à ladite composition.

31. Utilisation selon la revendication 29, comprenant en outre la mise en forme de ladite composition formée rigide après ladite application, pour conformer ladite composition aux caractères dudit site.

32. Utilisation selon la revendication 29, où ledit matériau osseux comprend de la poudre d'os déminéralisé.

33. Utilisation selon la revendication 32, où ledit matériau osseux comprend en outre des éclats d'os déminéralisé.

34. Utilisation selon la revendication 29, où ladite composition formée rigide comprend en outre un matériau de construction d'os.

35. Utilisation selon la revendication 34, où ladite composition formée rigide comprend en outre un matériau céramique contenant du calcium.

36. Utilisation selon la revendication 34, où ledit matériau de construction d'os comprend une protéine morphogénique osseuse, un facteur de croissance ou des mélanges de ceux-ci.

37. Utilisation selon la revendication 29, où ladite forme est un patch, un feuillet, un bloc, un anneau, un disque ou une préforme spécifique du site.

38. Utilisation selon la revendication 29, où ledit matériau osseux et ledit os déminéralisé dénaturé proviennent du même sujet.

39. Utilisation selon la revendication 29, comprenant en outre le chauffage de ladite composition osseuse formée rigide.

40. Utilisation selon la revendication 39, où ledit chauffage comprend l'utilisation d'un pack chauffant.
